# EUROPEAN PATENT APPLICATION

(11) **EP 1 174 498 A2**
(43) Date of publication of application: **23.01.2002**
(21) Application number: 01112430.2
(22) Date of filing: 22.05.2001
(51) Int. Cl.: C12N 9/64, C07K 14/745

(54) **Blood coagulation factor IX-activating protein and antibody thereto**

(30) Priority: 24.05.2000 JP 2000153096
(71) Applicant: Riken, Wako-shi, Saitama 351-0198 (JP)
(72) Inventor: Kaibara, Makota, Wako-shi, Saitama 351-0198 (JP); Iwata, Hiroki, Wako-shi, Saitama 351-0198 (JP); Takio, Koji, Wako-shi, Saitama 351-0198 (JP); Dohmae, Naoshi, Wako-shi, Saitama 351-0198 (JP)
(74) Representative: Sternagel, Fleischer, Godemeyer & Partner Patentanwälte

(57) **Abstract**

A purpose of the present invention is to clarify the mechanism of the intrinsic clotting system which is induced by activation of blood coagulation factor IX by erythrocyte membrane, and to clarify the structure of a factor which activates blood coagulation factor IX by isolating and identifying the factor.

The present invention provides a blood coagulation factor IX-activating protein derived from a mammal, having the following properties:
(1) the protein acts on blood coagulation factor IX to activate said factor;
(2) the activity of the protein is inhibited in the presence of an α1-protease inhibitor or soybean trypsin inhibitor;
(3) the protein is present in erythrocyte membrane;
(4) the protein has a molecular weight of approximately 29 kDa as measured by SDS-PAGE.

Sequence Listing
<110> RIKEN
<120> A blood coagulation factor activating protein and an
antibody thereof
<130> FA1128A/US
<160> 3
<210> 1
<211> 17
<212> PRT
<213> human
<220>
<222> 1
<223> Unknown amino acid
<220>
<222> 2
<223> Unknown amino acid
<400> 1
Xaa Xaa Pro Gln Gly Asp Ala Ala Gln Lys Thr Asp Thr Ser His His
1               5                   10                  15
Asp
<210> 2
<211> 18
<212> PRT
<213> human
<400> 2
Ile Val Gly Gly Arg Arg Ala Arg Pro His Ala Trp Pro Phe Met Val
1               5                   10                  15
Ser Leu
<210> 3
<211> 218
<212> PRT
<213> human
<220>
<222> 218
<223> Unknown amino acid
<400> 3

Ile Val Gly Gly Arg Arg Ala Arg Pro His Ala Trp Pro Phe Met Val
1               5                   10                  15
Ser Leu Gln Leu Arg Gly Gly Phe Phe Cys Gly Ala Thr Leu Ile Ala
                20                  25                  30
Pro Asn Phe Val Met Ser Ala Ala His Cys Val Ala Asn Val Asn Val
            35                  40                  45
Arg Ala Val Arg Val Val Leu Gly Ala His Asn Leu Ser Arg Arg Glu
    50                  55                  60
Pro Thr Arg Gln Val Phe Ala Val Gln Arg Ile Phe Glu Asn Gly Tyr
65                  70                  75                  80
Asp Pro Val Asn Leu Leu Asn Asp Ile Val Ile Leu Gln Leu Asn Gly
                85                  90                  95
Ser Ala Thr Ile Asn Ala Asn Val Gln Val Ala Gln Leu Pro Ala Gln
            100                 105                 110
Gly Arg Arg Leu Gly Asn Gly Val Gln Cys Leu Ala Met Gly Trp Gly
        115                 120                 125
Leu Leu Gly Arg Asn Arg Gly Ile Ala Ser Val Leu Gln Glu Leu Asn
    130                 135                 140
Val Thr Val Val Thr Ser Leu Cys Arg Arg Ser Asn Val Cys Thr Leu
145                 150                 155                 160
Val Arg Gly Arg Gln Ala Gly Val Cys Phe Gly Asp Ser Gly Ser Pro
                165                 170                 175
Leu Val Cys Asn Gly Leu Ile His Gly Ile Ala Ser Phe Val Arg Gly
            180                 185                 190
Gly Cys Ala Ser Gly Leu Tyr Pro Asp Ala Phe Ala Pro Val Ala Gln
        195                 200                 205
Phe Val Asn Trp Ile Asp Ser Ile Ile Xaa
    210                 215

## Description

### TECHNICAL FIELD OF THE INVENTION

The present invention relates to a blood coagulation factor IX-activating protein derived from a mammal, more specifically to a blood coagulation factor IX-activating protein which is present in the erythrocyte membrane. The present invention also relates to an antibody and a labeled antibody which recognize the protein, as well as a medicine which comprises the protein, the antibody or the labeled antibody.

### BACKGROUND ART

Blood is fluid when it circulates within a blood vessel. However, once blood is taken out of the blood vessel (for example, transferred into a test tube), it eventually loses its fluidity. This is because fibrinogen in the blood plasma is converted into fibrin to form a fibrin network, so that the whole blood gels. This phenomenon is called blood coagulation. A blood clot formed by blood coagulation entraps blood cells such as erythrocytes and leukocytes in the fibrin network. When a sufficient number of blood platelets with normal activity are present, the blood clot shrinks while extruding serum. This phenomenon is called clot retraction. When endothelial cells of the blood vessel wall fall away to a certain degree, and intravascular subcutaneous tissue comes into contact with blood, blood platelets adhere and coagulate to the subendothelial tissue and blood coagulation occurs, thereby forming a clot. A morbid condition, in which microthrombus occurs frequently in the blood vessels of the whole body, is referred to as disseminated intravascular clotting.

Blood coagulation, in which a factor in the tissue (especially, tissue thromboplastin) is involved, is called an extrinsic clotting system; and blood coagulation in which no factor is involved is called an intrinsic clotting system. The intrinsic clotting system begins when blood coagulation factor XII in the plasma comes into contact with the surface of a negatively charged solid phase(for example, glass). When factor XII is adsorbed onto the surface, limited proteolysis of the factor occurs and the factor is converted to activated factor XII (XIIa) which is an active protease. Factor XIIa converts factor XI, by limited proteolysis of factor XI, to an activated factor XI (XIa) which is an active protease. Factor XIa converts factor IX to activated factor IX (IXa) by limited proteolysis. Further, factor IXa activates factor X to form Xa. Thrombin is formed from prothrombin by the action of Xa. Finally, the thrombin converts fibrinogen to fibrin by limited proteolysis, terminating blood coagulation.

So far, it is known that a factor, which activates factor IX, is activated factor XI (XIa) as described above. In addition to this fact, the presence of an intrinsic coagulation reaction has been reported as the result from measurements and biochemical analyses, which were performed using rheological measurement systems developed for measuring blood clot and clot formation in stagnated blood stream *in vitro.* This reaction is caused by activation of blood coagulation factor IX by the erythrocyte membrane [Kawakami S, et al., Rheological approach to the analysis of blood coagulation in endothelial cell-coated tubes: Activation of the intrinsic coagulation reaction on the erythrocyte surface. Biorheology 32: 521-536, 1995; and Makoto Kaibara, et al., : Frontier Research on Circulation System Dynamics (report on research performed under a grant from the Science and Technology Agency, 1998), edited by Okayama New Technology Promotion Foundation, pp. 43-54, 1999].

However, the precise mechanism of this reaction remains unknown, and any factor which activates blood coagulation factor IX have not yet been identified.

### SUMMARY OF THE INVENTION

An object of the present invention is to clarify the mechanism of the intrinsic coagulation reaction which is caused by activation of blood coagulation factor IX by the erythrocyte membrane. Another object of the present invention is to isolate and identify a blood coagulation factor IX-activating factor to reveal the structure of the factor. Still another object of the present invention is to provide means for diagnosis, prevention and/or treatment of diseases associated with blood coagulation such as thrombosis using the factor or the antibodies thereto.

As a result of intensive studies to achieve the above-mentioned objects, the present inventors have succeeded in extracting and purifying a factor IX-activating protein which is expected to be present in the erythrocyte membrane, and determining an amino acid sequence thereof, thereby identifying the factor IX-activating protein (an enzyme protein).

Moreover, the present inventors have focused on the relation of some rheological factors involved in activation of blood coagulation factor IX, to aggregation of erythrocytes, and studied effects of shear rate on the activation of factor IX by erythrocyte membrane and on the time of onset of coagulation. As a result, the present inventors have found the activation of factor IX by erythrocytes was involved in formation of an aggregation structure of erythrocytes. Furthermore, the present inventors have focused on hematocrit (erythrocyte concentration) and studied an effect of hematocrit on the activation of factor IX by erythrocyte membrane and on the time of onset of coagulation. As a result, the present inventors have found that hematocrit was involved in activation of factor IX by erythrocytes.

Furthermore, in order to clarify the relation between hemodynamics of blood stream and formation of thrombus, the present inventors have constructed a device for examining in vitro dynamics of thrombus formation in an aneurysmal flow-pass model, and conducted fundamental experiments. As a result, it has been found that clot was formed only inside an aneurysm in which blood stagnation occurred. Further, in order to study whether there are differences in blood coagulation and activation of factor IX by erythrocytes between individuals, the present inventors have examined the time of onset of coagulation and the activation of factor IX by erythrocyte membrane of each of normal subjects, and diabetics and normal pregnant women who are apt to form thrombus. As a result, it has been found that the blood of normal subjects significantly differed from the blood of the subjects having thrombus forming tendency in respect of the time of onset of coagulation and the activation rate of factor IX by erythrocyte membranes.

The present invention has been completed based on these findings.

Thus, the present invention provides a blood coagulation factor IX-activating protein derived from a mammal, having the following properties:
(1) the protein acts on blood coagulation factor IX to activate said factor;
(2) the activity of the protein is inhibited in the presence of an α1-protease inhibitor or soybean trypsin inhibitor;
(3) the protein is present in erythrocyte membrane;
(4) the protein has a molecular weight of approximately 29 kDa as measured by SDS-PAGE.

The blood coagulation factor IX-activating protein of the present invention preferably cleaves the amino acid sequence of the blood coagulation factor IX between 140th threonine and 141th serine, between 181th valine and 182th valine, and between 182th valine and 183th glycin.

The blood coagulation factor IX-activating protein of the present invention preferably comprises the following partial amino acid sequence:

Ile-Val-Gly-Gly-Arg-Arg-Ala-Arg-Pro-His-Ala-Trp-Pro-Phe-
Met-Val-Ser-Leu

The blood coagulation factor IX-activating protein of the present invention is preferably derived from human.

The blood coagulation factor IX-activating protein of the present invention is preferably purified by disrupting erythrocytes, extracting with a surfactant, and subjetcing the extract to anion exchange chromatography and heparin affinity chromatography.

According to another aspect of the present invention, there is provided a medicine which comprises a blood coagulation factor IX-activating protein of the present invention. The medicine is used, for example, for treatment and/or prevention of diseases associated with abnormal blood coagulation.

According to still another aspect of the present invention, there is provided an agent for activating blood coagulation factor IX which comprises a blood coagulation factor IX-activating protein of the present invention.

According to still another aspect of the present invention, there is provided an antibody or fragment thereof which recognizes a blood coagulation factor IX-activating protein of the present invention. The antibody is preferably a monoclonal antibody.

According to still another aspect of the present invention, there is provided a medicine which comprises an antibody or fragment thereof of the present invention. The medicine is used, for example, for treatment and/or prevention of diseases associated with abnormal blood coagulation.

According to still another aspect of the present invention, there is provided a labeled antibody or fragment thereof which recognizes a blood coagulation factor IX-activating protein of the present invention.

According to still another aspect of the present invention, there is provided a medicine which comprises a labeled antibody or fragment thereof of the present invention. The medicine is, for example, a diagnostic drug for diseases associated with blood coagulation.

According to still another aspect of the present invention, there is provided a method for detecting a blood coagulation factor IX-activating ability which comprises detecting or measuring a blood coagulation factor IX-activating protein of the present invention in a biological sample.

### BRIEF DESCRIPTION OF DRAWINGS

Figure 1 shows the protocol for the purification of factor IX-activating protein of erythrocyte membranes.
Figure 2 shows results of separation by chromatography for purifying factor IX-activating protein. Figure 2(a) shows the absorbance of the eluates from the second heparin affinity chromatography, and Figure 2(b) shows the presence or absence of activation of factor IX in each fraction from the eluates.
Figure 3 shows results when extracts were allowed to react with factor IX after chromatography on a soybean trypsin inhibitor bound agarose column, followed by silver-staining.
Figure 4 shows results of analysis and database search conducted for amino acid sequences of the extract. Figure 4 (a) shows results of analysis of a complex of the extract and α₁ protease inhibitor; (1) indicates α₁ protease inhibitor (SEQ ID NO: 1), (2) indicates the amino acid sequence of the extract (SEQ ID NO: 2); Figure 4 (b) shows the amino acid sequence (SEQ ID NO: 3) from human leukocyte. An underlined part represents the site (2) in panel (a).
Figure 5 shows results of examining the effect of shear rate on blood coagulation of RBCs/PFP system. Figure 5 (a) shows increases in viscosity along with coagulation of RBCs/PFP samples. The increases were measured using a cone-plate viscometer at a constant shear rate. Figure 5 (b) shows the relation between the time of onset of coagulation and the shear rate.
Figure 6 shows the effect of shear rate on activation of factor IX by erythrocyte membranes which was measured by SDS-PAGE. Shear rate: (a) 0.5sec⁻¹, (b) 50sec⁻¹.
Figure 7 (a) shows the effect of hematocrit on the time of onset of coagulation of whole blood or of RBCs/PFP. The figure also shows the relation between the time of onset of coagulation of PRP and the number of blood platelets. Figure 7 (b) shows the effect of hematocrit on the activation of factor IX by erythrocyte membrane as measured by SDS-PAGE.
Figure 8 shows changes with time in the activation of factor IX by erythrocyte membranes as measured by SDS-PAGE. Figure 8(a) shows a change for erythrocytes of normal subjects; Figure 8(b) shows a change for erythrocytes of diabetics.
Figure 9 shows a change in impedance (Z) and a change in logarithmic damping factor (LDF) in the course of blood coagulation.
   ○ denotes LDF; ● denotes Z.
Figure 10 shows comparison of cleavage sites of factor IX by an enzyme extracted by the present invention, elastase from leukocytes, and activated factor XI (XIa).
Figure 11 shows results of evaluation of an enzyme activity on erythrocyte membranes by fluoromicroscopic observation.
Figure 12 shows the relation between the fluorescent intensity and the time of onset of coagulation in the sample (RBCs/PEP)free from platelets and leukocytes.

### DETAILED DESCRIPTION OF THE INVENTION

Embodiments and methods for implementing the present invention will be given in detail as follows.

### A. Blood coagulation factor IX-activating protein

The blood coagulation factor IX-activating protein derived from a mammal of the present invention, is characterized by the following properties:
(1) the protein acts on blood coagulation factor IX to activate said factor;
(2) the activity of the protein is inhibited in the presence of an α1-protease inhibitor or soybean trypsin inhibitor;
(3) the protein is present in erythrocyte membrane;
(4) the protein has a molecular weight of approximately 29 kDa as measured by SDS-PAGE.

In the present invention, types of mammal include, but are not specifically limited to, any mammal including human (e.g., human, monkey, mouse, rat, hamster, bovine, pig, goat, sheep, dog, and cat). A preferable mammal is a human.

Blood coagulation factor IX is a factor which is referred to as antihemophilic factor B, Christmas factor or plasma thromboplastin (PTC). Blood coagulation factor IX is a single-stranded vitamin K-dependent serine protease having a sugar content of 17% (human) or 26% (bovine), and a molecular weight of 57,000 (human) or 55,000 (bovine). Factor IX is produced in the liver and is deficient in hemophiliac B. An activated factor XI cleaves an Arg-Ala bond of factor IX into an inactive form of a double stranded intermediate that contains a S-S cross-link. Then, activated factor XI further hydrolyzes the Arg-Val bond into H chain of a molecular weight of 27,000 and L chain of a molecular weight of 17000 in the presence of Ca²⁺. At this time, activated peptides (which have a molecular weight of 11,000 and sugar content of 70%) are liberated from the N terminus of the H chain. Factor IX is also activated by factor VII and tissue thromboplastin in the presence of Ca²⁺. In addition, activated factor IX cleaves an Arg-Ile bond of H chain of factor X in the presence of Ca²⁺ and phospholipid into activated factor X.

The blood coagulation factor IX-activating protein of the present invention is different from these known activators of blood coagulation factor IX as described above.

The activity of the blood coagulation factor IX-activating protein of the present invention is inhibited in the presence of an. ₁-protease inhibitor or a soybean trypsin inhibitor. This suggests that the blood coagulation factor IX-activating protein of the present invention activates factor IX by exhibiting protease activity.

The blood coagulation factor IX-activating protein of the present invention is present in the erythrocyte membrane. Erythrocyte membrane, which means a plasma membrane of erythrocytes, is substantially identical to a residue or ghost remaining after hemolysis, since erythrocytes contain no other membrane structure and granular structure.

The blood coagulation factor IX-activating protein of the present invention has a molecular weight of approximately 29 kDa as measured by SDS-PAGE.

The blood coagulation factor IX-activating protein of the present invention is most likely a type of elastase. Elastase is a protease whose specific substrate is elastin. It was first purified from the bovine pancreas, and pancreatic elastase and leukocyte elastase have been known. Elastase well hydrolyzes protein substrates other than elastin. Elastase has a molecular weight of 28,900, and is classified as a serine protease.

The blood coagulation factor IX-activating protein of the present invention can be purified by disrupting erythrocytes, extracting with a surfactant, followed by anion exchange chromatography and heparin affinity chromatography.

Erythrocytes can be disrupted by washing them with an appropriate buffer and suspending them in distilled water to lyse the blood. Lipid components in erythrocyte membrane are extracted with a chloroform/methanol mixture, and then protein components are extracted with a buffer containing a surfactant, such as Tween 20. After removal of insoluble components by centrifugation, the factor IX activating ability in the extract is examined.

Activation of factor IX can be measured as follows. Erythrocytes are washed three times with HEPES buffer (50mM HEPES, 115mM NaCl, 5mM KCl, 0.1%(w/v) glucose, pH7.4). Pure factor IX is dissolved in HEPES buffer and mixed with the washed erythrocytes (hematocrit: 40%). Next, CaCl₂ is added to the suspension, the suspension is incubated at 37°C for 30 minutes, and then EDTA is added to the suspension to stop the reaction. Subsequently, erythrocytes are completely removed by centrifuging the suspension, and then the supernatant is subjected to SDS-PAGE to examine the presence of activated coagulation factors. Here, 7.5 to 15% gradient gel is used and electrophoresis is performed with a constant current of 20mA in the presence of 0.1% SDS. A protein band is stained with Coomassie brilliant blue for 3 hours. When activated factor IX is detected by SDS-PAGE, immunostaining can be performed to further confirm that the obtained band is an active form of blood coagulation factor IX. Procedures for immunostaining include buffering the gel after electrophoresis, and transferring the gel to a PVDF membrane (Polyvinylidene difluoride). The PVDF membrane to which proteins are transferred is washed, followed by blocking and treatment with a specific antiserum (rabbit anti-human factor IX antiserum). After washing the membrane, PVDF membrane is treated with peroxidase-labeled goat anti-rabbit IgG antibodies, thereby labeling coagulation factors. The thus obtained PVDF membrane can be stained with a solution prepared by adding 30% (v/v) H₂O₂ to TBS (Tris buffer) containing DAB (3,3'-diaminobenzidine).

Active fractions extracted with a surfactant are subjected to anion exchange chromatography using a column buffered with a buffer containing Tween 20. An example of the column is Q-Sepharose column. The column is subjected to an elution treatment with a buffer having an appropriate salt concentration, to collect fractions. Among the resulting fractions, active fractions can be subjected to heparin affinity chromatography and fractionated by elution with a gradient of appropriate salt concentration in a buffer. The activity of each fraction is measured as described above.

Fractions containing the protein of the present invention having the desired activity can be isolated by repeating the above-mentioned anion exchange chromatography and heparin affinity chromatography in combination in a suitable number of times.

### B. Antibody recognizing blood coagulation factor IX-activating protein

Since a factor IX-activating protein that is present in erythrocyte membrane is identified according to the present invention, antibodies to this protein can be prepared. Preparation of such antibodies is useful as a prophylactic or therapeutic agent against thrombus-formation caused by activation of factor IX.

The present invention also relates to an antibody which recognizes the blood coagulation factor IX-activating protein. The antibody of the present invention may be a polyclonal antibody or monoclonal antibody, and can be prepared by standard techniques.

For example, a polyclonal antibody recognizing the blood coagulation factor IX-activating protein of the present invention can be obtained by immunizing a mammal with the blood coagulation factor IX-activating protein of the present invention as an antigen, collecting the blood from the mammal, separating and purifying antibodies from the collected blood. For example, mammals such as mouse, hamster, guinea pig, chicken, rat, rabbit, dog, goat, sheep and bovine can be immunized. Immunization can be performed by administering an antigen once or more according to a standard immunization method, such as that of W. H. Newsome as described in J. ASSOC. OFF. ANAL. CHEM 70(6) 1025-1027 (1987).

Preferably, an antigen is administered two or three times at an interval of 7 to 30 days, particularly 12 to 16 days. A dose of about 0.05 to 2mg of antigen can be used. The route of administration is not specifically limited, and can be appropriately selected from subcutaneous, intracutaneous, intraperitoneal, intravenous, and intramuscular administrations. Administration by intravenous, intraperitoneal, or subcutaneous injection is preferred. Antigens may be used by dissolving in an appropriate buffer containing a general adjuvant, such as complete Freund's adjuvant, RAS [MPL (Monophosphoryl Lipid A)+TDM (Synthetic Trehalose Dicorynomycolate)+CWS(Cell Wall Skeleton) Adjuvant system], aluminum hydroxide. There may be cases where the above adjuvant may not be used depending on administration routes, conditions or the like. The term "adjuvant" used herein means a substance which enhances an immune reaction with an antigen nonspecifically when administered together with the antigen.

An Immunized animal is reared for 0.5 to 4 months, a small amount of the serum from the mammal is taken from an ear vein and the like, and then the antibody titer is measured. When the antibody titer starts to elevate, antigen is administered a suitable number of times where necessary. For example, booster immunization is performed using 100. g to 1000. g of the antigen. Blood is collected by a standard method from an immunized mammal 1 to 2 months after the final administration. The collected blood is separated and purified by standard techniques including centrifugation, precipitation using ammonium sulfate or polyethylene glycol, and chromatography, such as gel filtration chromatography, ion exchange chromatography and affinity chromatography. As a result, polyclonal antibodies which recognize the blood coagulation factor IX-activating protein of the present invention can be obtained as a polyclonal anti-serum. The complement system can be inactivated by treating the anti-serum, for example, at 56°C for 30 minutes.

The globulin type of the monoclonal antibody which recognizes the blood coagulation factor IX-activating protein of the present invention includes, but is not specifically limited to, IgG, IgM, IgA, IgE, and IgD. Moreover, the antibody of the present invention is preferably a humanized antibody or human antibody.

A cell line producing the monoclonal antibody of the present invention is not specifically limited. For example,a hybridoma can be obtained by cell fusion of an antibody-producing cell and a myeloma cell line. Hybridomas which produce the monoclonal antibodies of the present invention can be obtained by the cell fusion method as described below.

Examples of antibody-producing cells used herein include those obtained from immunized animals, such as spleen cells, lymph gland cells, and B cells. Examples of antigens used herein include the blood coagulation factor IX-activating protein of the present invention or a partial peptide thereof. Animals to be immunized include mouse and rat. Antigen is administered to these animals according to standard techniques. For example, a suspension or emulsion which contains an adjuvant such as complete or incomplete Freund's adjuvant, and the blood coagulation factor IX-activating protein as an antigen, is prepared. The prepared suspension or emulsion can be administered intravenously, subcutaneously, intracutaneously, or intraperitoneally to an animal several times for immunization. Then, antibody-producing cells (e.g., spleen cells) can be collected from the immunized animal, followed by fusion of the collected cell with a myeloma cell by a known method (G.Kohler et al., Nature, 256 495, 1975), thereby preparing a hybridoma.

Examples of myeloma cell lines used for cell fusion include the cell lines P3X63Ag8, P3U1 and Sp2/0 for mouse. In the cell fusion, a fusion promoting agent such as polyethylene glycol or Sendai virus may be used. In the selection of hybridomas after cell fusion, a hypoxanthine/aminopterin/thymidine (HAT) medium is used according to usual techniques.

Hybridomas obtained by cell fusion are cloned by using methods such as limiting dilution. Further, a cell line producing a monoclonal antibody which specifically recognizes the blood coagulation factor IX-activating protein of the present invention, can be obtained by screening by an enzyme immunoassay using the blood coagulation factor IX-activating protein.

To produce a monoclonal antibody of interest from the hybridomas thus obtained, the hybridomas are cultured by a normal cell culture or ascite-formation method, and then the monoclonal antibodies are purified from culture supernatants or ascites. Monoclonal antibodies can be purified from culture supernatants or ascites by a normal method. For example, ammonium sulfate fractionation, gel filtration, ion exchange chromatography, affinity chromatography and the like, can be used appropriately in combination.

Examples of immunoassay for the blood coagulation factor IX-activating protein using the monoclonal antibodies of the present invention include enzyme immunoassay, radioimmunoassay, fluorescence immunoassay, and luminescent immunoassay. An enzyme immunoassay, so-called sandwich enzyme immunoassay, can be performed using a monoclonal antibody-bound insoluble carrier, which is prepared by binding the monoclonal antibody to an insoluble carrier.

The present invention also encompasses fragments of variety antibodies described above. Such fragments of antibodies include F(ab')2 fragment and Fab'fragment.

### C. Labeled antibodies which recognize blood coagulation factor IX-activating protein

The present invention also relates to a labeled antibody which recognizes a blood coagulation factor IX-activating protein. The antibodies of the present invention prepared as described above can be labeled and used. Preparation of such a labeled antibody allows determination of an enzyme protein level on the surface of erythrocyte membrane. This can also lead to the possible development of diagnostic drugs for thrombosis caused by activation of factor IX.

The type of label for antibodies and labeling method can be appropriately selected from those known by persons skilled in the art.

When enzymes are used as labels, examples of such labels include horseradish peroxidase, alkaline phosphatase, glucose oxidase, .-galactosidase, glucoamylase, carbonic anhydrase, acetylcholine esterase, lysozyme, malate dehydrogenase, and glucose-6-phophate dehydrogenase. Examples of methods for labeling the antibody or fragment thereof (F(ab')2 fragment, Fab' fragment, etc.) with these enzymes, include a method in which sugar chains of the enzyme are oxidized with periodic acid, and amino acids such as those of the antibody are bound to the generated aldehyde groups; and a method in which a maleimide group or a pyridyl sulfide group or the like is introduced into an enzyme and the enzyme is bound with a thiol group present in Fab' fragments of the antibody.

When an enzyme is used as a label, test samples and labeled antibodies are incubated, free labeled antibodies are removed by washing, and then the substrate of the above labeled enzyme is allowed to react to measure the reaction based on color development, thereby detecting the labeled antibodies. For example, when the antibody is labeled with peroxidase, the use of hydrogen peroxide as a substrate, and diaminobenzidine or o-phenylenediamine as a chromagenic reagent in combination gives a brown or yellow color. When the antibody is labeled with glucose oxidase, for example, 2,2'-acido-di-(3-ethylbenzothiazolin-6-sulfonic acid (ABTS)) may be used as a substrate.

When a fluorescent dye is used as a label, the antibody or fragment thereof of the present invention can be labeled with, for example, a fluorescent dye such as FITC (fluorescein isothiocyanate) and TRITC (tetramethyl rhodamine B isothiocyanate). Binding of the antibody or fragment thereof of the present invention to a fluorescent dye can be performed according to a standard method.

When a chromagenic label is used as a label, colloidal metals and colored latex can be used as labels. Typical examples of colloidal metals include metal colloidal particles which are dispersed particles of each of gold sol, silver sol, selenium sol, tellurium sol, platinum sol and the like. The size of a colloidal metal particle is generally of a diameter of about 3 to 60nm. A typical example of colored latex is a synthetic latex such as polystyrene latex colored with a pigment such as red or blue. As latex, natural latex such as natural rubber latex can be used. The size of colored latex can be selected from a diameter range of approximately several tens nm to several hundreds nm. Commercial chromogenic labels can also be used as they are. These commercial products themselves can be processed or manufactured by a known method if necessary.

Binding of the antibody or fragment thereof of the present invention to a chromogenic label can be performed according to standard techniques. For example, when gold colloidal particles, which are dispersed particles of gold sol, are used as a chromogenic label, antibodies and gold sol can be physically bound to each other by mixing the two at room temperature.

In addition to the above described labels, affinity labels (e.g., biotin) or isotope labels (e.g., ¹²⁶I) or the like can also be used as labels.

Analytical methods using the labeled antibodies of the present invention, such as the enzyme antibody technique, immuno tissue-staining method, immunoblotting method, direct fluorescent antibody technique or indirect fluorescent antibody technique, can be performed according to standard techniques known by persons skilled in the art. The experimental conditions can also be appropriately selected by persons skilled in the art.

The scope of the invention encompasses the labeled antibodies or fragments thereof of the above-mentioned antibodies or fragments thereof, which recognize the blood coagulation factor IX-activating protein of the present invention. Furthermore, the scope of the invention also encompasses a reactive reagent, which contains the above described labeled antibody or fragment thereof in a buffer, such as PBS (phosphate buffer). Such a reagent may contain an additive, such as gelatin known to persons skilled in the art. The scope of the invention further encompasses various detection (measurement) kits which contain the reactive agent of the present invention as a component, as well as other antibodies and/or various measuring instruments, buffers, reagents, and the like depending on the desired detection technique.

### C. Medicine which comprises the blood coagulation factor-activating protein, antibody, or labeled antibody of the present invention

The blood coagulation factor-activating protein of the present invention can act on blood coagulation factor IX to activate the factor and promote blood coagulation. Thus, the blood coagulation factor-activating protein of the present invention can be used as a blood coagulation promoting agent, for example by administering to a site that needs the blood coagulating action. When used as a hemostat for suppressing defluxion of blood from vessels caused by an external injury and the like, the blood coagulation factor-activating protein of the present invention is made into a dosage form of powder or liquid, and is directly sprinkled over the bleeding site, thereby arresting the bleeding. When used as an agent for arresting the blood stream by vascular occlusion, the blood coagulation factor-activating protein of the present invention is prepared as a solution, and the solution is administered through an instrument for angiostomy including a cannula, catheter, or syringe. Upon administration, the tip of such an instrument is inserted to a desired site of the blood vessel to be occluded, and then the solution is administered via the instrument. The dose and time for administration of the agent for blood occlusion can be appropriately selected depending on the width and site of a vessel that needs blood occlusion.

Examples of pharmaceutical formulations which comprises the blood coagulation factor-activating protein of the present invention as an active ingredient include pulverulent solid formulations and liquid formulations. The blood coagulation factor-activating protein of the present invention can be formulated in the form of a pharmaceutical composition, which contains a pharmacologically acceptable carrier, depending on the administration method and the dosage form, in addition to the active ingredient. When the medicine of the present invention is used as a liquid formulation, the blood coagulation factor-activating protein of the present invention as an active ingredient is preferably dissolved or suspended in an appropriate pharmacologically acceptable solvent such as physiological saline, glycerol and phosphate buffer at an appropriate concentration. A solid formulation, for example a pulverulent solid formulation, may be a solid consisting only of the above pharmacologically acceptable salt, or may be the product obtained by exsiccating the above liquid formulation.

It is considered that the antibody of the present invention can suppress blood coagulation by blocking *in vivo* the action of the blood coagulation factor-activating protein of the present invention. That is, the antibody of the present invention is useful as a prophylactic or therapeutic agent against diseases (e.g., thrombosis) associated with excessive blood coagulation. The antibody of the present invention is administered at a dose and through a route appropriate for the type and severity of a disease to be treated. Such dose and route can be appropriately determined by persons skilled in the art. A preferable route of administration is, for example, subcutaneous injection, intramuscular injection, or intravenous injection. Moreover, the antibody of the present invention has an advantage that it stops blood coagulation by directly binding to the factor IX-activating protein present on the surface of an erythrocyte membrane, thus the antibody causes no bleeding tendency even if the protein is excessively present in the plasma.

Examples of the medicine containing the antibody of the present invention includes a pharmaceutical composition which comprises a therapeutically effective amount of the antibody of the present invention together with a pharmaceutically acceptable adjuvant. The pharmaceutical composition may also contain optional ingredients, such as a diluent, carrier, preservative, emulsifier, antioxidant and/or stabilizer. A pharmaceutically acceptable adjuvant is known among persons skilled in the art and described in detail in Remington's Pharmaceutical Sciences, 18^{th} ed., A. R. Gennaro eds., Mack, Easton, PA (1990).

The dose of the medicine containing the blood coagulation factor-activating protein of the present invention or the antibody of the present invention can be appropriately selected depending on a dosage form, a type of an active ingredient, an animal to be administered (e.g., human, mouse, rat, bovine or horse), and a purpose of administration. For example, the dose can be selected within a the range of about 1 mg to 500 mg/kg body weight. The number of administration can be changed as described above, and properly selected within a range of 1 to 4 times a week to 1 to 3 times a day.

### E. A method for detecting a blood coagulation factor IX-activating ability

According to the present invention, there is provided a method for detecting a blood coagulation factor IX-activating ability which comprises detecting or measuring a blood coagulation factor IX-activating protein of the present invention in a biological sample.

A method for detecting or measuring a blood coagulation factor IX-activating protein of the present invention is not particularly limited, and the analysis can be carried out by a method known to persons skilled in the art using a synthetic substrate. Alternatively, the blood coagulation factor IX-activating protein of the present invention can be detected or measured by using the antibody described herein. By detecting a blood coagulation factor IX-activating ability, a disease associated with blood coagulation (for example, thrombosis) can be diagnosed.

The present invention will now be described in more detail by the following examples, but it is intended that the scope of the present invention is not limited to those examples.

### Examples

### Example 1:

### A. Methods

### (1) Analysis of factor IX-activating reaction

### ① Blood sample

In all experiments, blood from normal humans, diabetics (glycated hemoglobin > 7.0), and normal pregnant women (gestational weeks: 31.3 ±8.4) were used. Blood was collected via the cubital vein. Then, the blood, and an anti-coagulant, an aqueous solution of 3.8% sodium citrate, were mixed at a proportion of 9:1 (blood:aqueous solution volume/volume) in a polypropylene tube (inactive to coagulation) to obtain a non-coagulable mixture. Erythrocytes (RBCs) were obtained by centrifuging the blood at 1,200 x g for 10 minutes and removing the supernatant. The supernatant was further centrifuged at 16,000 x g to prepare platelet free plasma (PFP). In addition, a sample mixture of erythrocytes and PFP (RBCs/PFP) was prepared. Erythrocytes were washed with HEPES buffer (50mM HEPES, 115mM NaCl, 5mM KCl, 0.1% (w/v) glucose, pH7.4) where necessary.

### ② Measurement of activation of factor IX by an erythrocyte membrane

25 *µ*g of pure factor IX (Enzyme Res. Lab.) was dissolved in 50 *µ*l of HEPES buffer, and was mixed with washed erythrocytes (hematocrit: about 40%). 10 *µ*l of 26 mM CaCl₂ was added to the suspension, and the mixture was incubated at 37°C for 30 minutes, and then 30 *µ*l of 0.1 M EDTA was added to the suspension to stop the reaction. The suspension was centrifuged to completely remove erythrocytes, and the supernatant was subjected to SDS-PAGE and Western blot analysis to examine whether the coagulation factor was activated or not. 7.5 to 15% gradient gel was used, and electrophoresis was performed at a constant current of 20 mA in the presence of 0.1% SDS. Protein bands were stained for 3 hours with a 50% ethanol(v/v)/7% acetic acid solution containing 0.25%(w/v)Coomassie brilliant blue R-250 (CBB). When activated factor IX was detected by SDS-PAGE, immunostaining was performed to confirm that the resulting band was of activated coagulation factor IX. In the immunostaining procedures, the gel subjected to electrophoresis was buffered using Towbin buffer (25mM Tris-HCl (pH 8.3), 192mM glycin, 20%(v/v) methanol, 0.1% SDS) for 15 minutes, followed by transferring to a polyvinylidene difluoride (PVDF) membrane under conditions of 15V and 150mA. PVDF on which proteins were transferred, was washed with 20mM Tris HCl (pH7.4) + 500mM NaCl buffer (TBS) for 30 minutes, followed by treatment with TBS containing 1%(w/v) bovine albumin for 30 minutes. Following washing with TBS, the PVDF membrane was treated with TBS containing 0.2%(v/v) specific anti-serum (i.e. rabbit anti-human factor IX antiserum). Following further washing with TBS, the PVDF was treated with 0.2%(v/v) peroxidase-labeled goat anti-rabbit IgG antibody to label a coagulation factor. The thus-obtained PVDF membrane was stained with a solution prepared by adding 10 *µ*l of 30%(v/v) H₂O₂ to TBS containing 0.05%(w/v) DAB (3,3'-diaminobenzidine).

### ③ Extraction of proteins from erythrocyte membrane

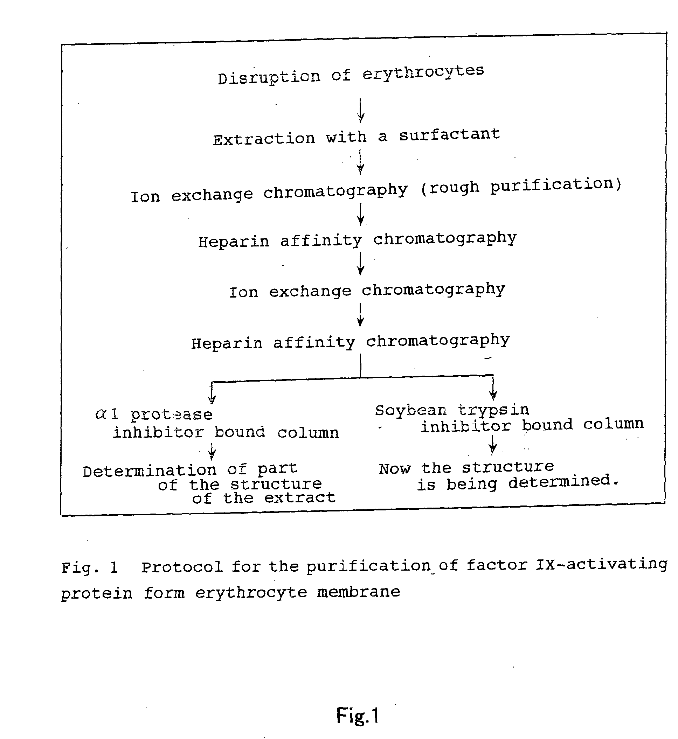

After washing with HEPES buffer solution, erythrocytes were suspended in distilled water, lysed and destroyed. Lipid in the erythrocyte membrane component was extracted with a chloroform-methanol mixture (2:1, V/V)(It was confirmed that lipid components did not activate factor IX). After the destroyed erythrocyte membrane was washed with 1% n-octyl-. -D-glucoside (OG), the protein component was progressively extracted overnight at 4°C using 50mM Tris-HCl buffer (pH7.4) containing Tween 20. After removal of insoluble components by centrifugation, the factor IX-activating ability of the extracts was examined. Fractions that activated factor IX were purified by column chromatography. Several methods were tried for purification. Figure 1 shows a protocol for purification that was finally employed. Details of this method are shown in the results section.

### ④ Structural analysis

2 *µ*g of a sample was subjected to SDS polyacrylamide gel electrophoresis, and transferred to a PVDF membrane, followed by staining with Coomassie dye. A band corresponding to 50 kDa was collected, and then the sequence was analyzed from the amino-terminus by an amino acid sequencer (477A, Perkin-Elmer) (Masutani C, Kusumoto R, Yamada A et al.: The XPV (xeroderma pigmentosum variant) gene encodes human DNA polymerase 7. Nature 399: 700-704, 1999).

To determine the internal amino acid sequence, the band stained with Coomassie dye and corresponding to 50 kDa, and a gel portion to which no sample had been applied, were collected and were immersed in 50 ml of a 100 mM Tris buffer containing 1 mM EDTA and 0.1% SDS buffer (pH 9.0), followed by digestion with 0.1 *µ*g of *Achromobacter* protease I (API/Lys-C) in each gel overnight at 37°C. The digested solution was separated with HPLC (where DEAE 5PW column with diameter 1mm x 20mm and Mightysil PR-18 mm column with diameter of 1mm x 50mm were connected in tandem) and fractionated. Elution was performed using two types of solution at a flow rate of 20 *µ*l. The resulting principal peaks were subjected to mass spectroscopy with a time-of-flight type mass spectrometer (reflex, Burker/matrix: 2-benzothiazole) provided with the matrix-supported laser elimination ionization method. Further, some samples were subjected to amino acid analysis, and the results were analyzed in the data bank, thereby determining the structure.

### (2) Measurement of the time of onset of blood coagulation

The time of onset of blood coagulation was measured using a damped oscillation rheometer (kaibara M et al., A new rheological method to measure fluidity change of blood during coagulation: Application to in vitro evaluation of anticoagulability of artificial materials. Biorheology, 22, 197-208, 1985). A blood sample was put into a polypropylene tube (3 cm long, 0.9 cm internal diameter), and changes in blood fluidity in the course of blood coagulation were measured. Using this device, a logarithmic damping factor (abbreviated as LDF) showing liquid fluidity is measured, and a time required for coagulation to start can be precisely determined based on a time point at which LDF value starts to change in the course of coagulation.

### (3) Shear rate dependent rheological measurement of activation of factor IX and coagulation by erythrocyte membranes

Activation of factor IX and coagulation processes by erythrocyte membrane were examined in a flow controlled at a constant shear rate using a cone-plate viscometer. Since anti-thrombosis of the stainless surface of the cone-plate of viscometer is poor, the surface was coated with segmented-polyurethane (SPU, Pellethane, Dow Chemicals, USA) to prepare an anti-thrombogenic surface (Kaibara M et al., Proliferation of endothelial cells on the plasma-treated segmented-polyurethane surface: Attempt of construction of a small caliber hybrid vascular graft and antithrombogenicity. Colloids and Surfaces, B: Biointerfaces, 19, 209-217, 2000). SPU was used after dissolving in a 1:1 mixed solvent of tetrahydroxyfuran and dimethylsiloxane at 1% concentration, casting over the cone-plate surface, and drying.

### (4) Measurement of thrombus formation in flow pass model for bosselation

Impedance measurement (Katsuyuki Sakamoto et al., electrical characteristics of flowing blood, Medical Engineering and Bioengineering, 16: 45-57, 1978; and Mamiko Fujii et al., Analysis of deformation and orientation of erythrocytes in flowing blood by measurement of electric resistance rate, the 47^{th} Rheology forum abstracts, pp.295-296, 1999) of the process of clot formation in a flow pass model for bosselation was performed using a LCR meter and a platinum electrode. For the purpose of comparison, a blood coagulation process was also measured using a damped oscillation rheometer.

### B. Results

### (1) Purification of factor IX-activating proteins

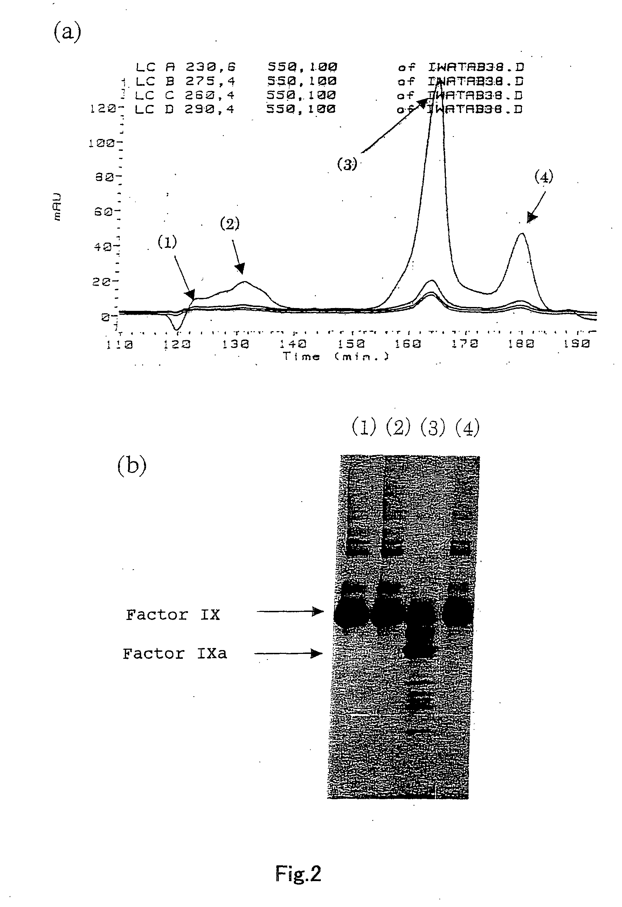

Procedures for purifying factor IX-activating proteins present in erythrocyte membrane as shown in Fig. 1 will be described in detail.
(i) Active fractions extracted with a surfactant were subjected to anion exchange chromatography using Q-Sepharose column (1.5 x 3 cm) buffered with buffer A (50 mM Tris-HCl buffer containing Tween 20). The column was washed with buffer A and eluted with buffer B (50 mM Tris HCl + 1.0M NaCl, pH 7.4). The absorbance of fractions was measured at wavelengths of 230, 260, 275, and 290 nm at a flow rate of 1.0ml/min. Then the fractions were aliquoted, and the factor IX-activating ability of each fraction was measured. Most of extracted fractions showed activity.
(ii) Eluate was desalted using a Sephadex G-25 column (1.5 x 3 cm) buffered with buffer A, and then subjected to affinity chromatography using a HiTrap Heparin column (1.5x3 cm) buffered with buffer A. After washing with buffer A, the column was eluted with NaCl linear gradient from 0 to 1.0 M. Activity was measured for each of the fractions.
(iii) Active fractions were desalted, and subjected to anion exchange chromatography on Q-sepharose again. After washing with buffer A, the column was eluted with NaCl linear gradient from 0 to 0.5M.
(iv) After activity measurement, active fractions were further separated using a HiTrap Heparin column buffered with buffer A. After washing with 50mM Tris-HCl, pH7.4, 0.3 M NaCl, the column was eluted with a NaCl linear gradient from 0.3 to 0.8 M. Absorbance was measured, and then fractions were fractionated at a flow rate of 0.7 ml/min (Fig. 2(a)). As a result from the measurement of the factor IX-activating ability of each of the fractions, strong activity was detected in fraction (3) (Fig. 2 (b)).

It was confirmed by SDS-PAGE that if α1-protease inhibitor (α1-PI) which is a serine protease inhibitor, or soybean trypsin inhibitor (SBTI) is added to a solution containing an extract of interest, the activation of factor IX by the extract is inhibited.

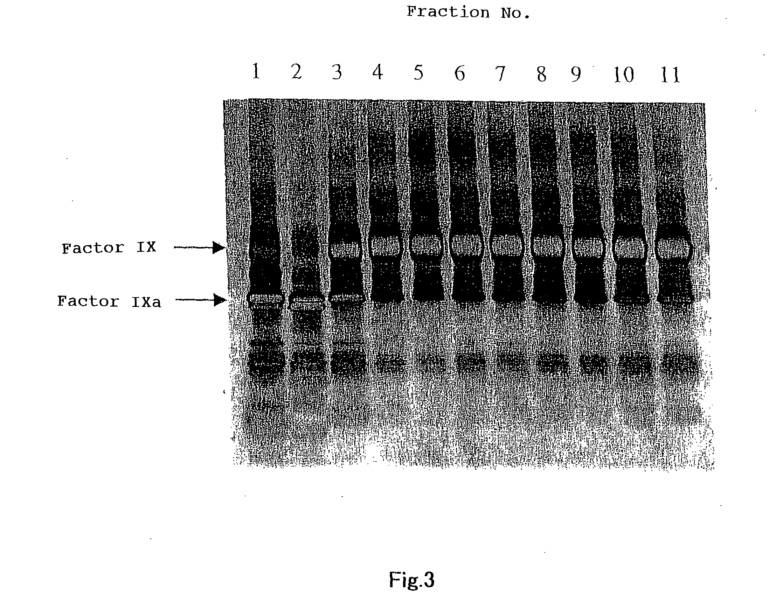

The solution containing the extract was applied to soybean trypsin inhibitor bound agarose column, and was eluted with a buffer. Then the proteins in the eluate were allowed to react with factor IX, followed by staining with silver. The first fraction showed strong activity, as shown in Fig. 3. Purification was confirmed based on the fact that the activation rate of factor IX corresponded to the amount of the enzyme protein (strength of bands).

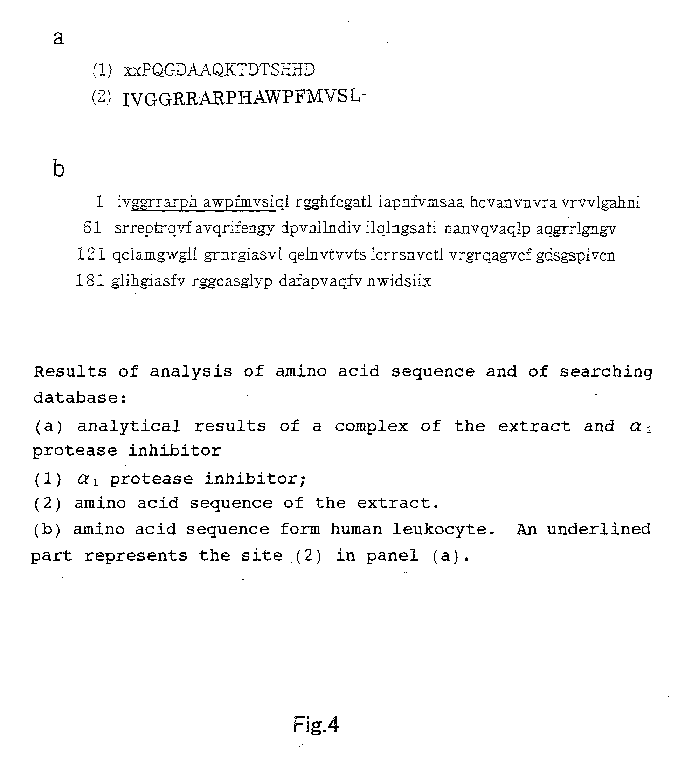

Since α₁ protease inhibitor and the extract formed a complex, a partial amino acid sequence of the extract from the complex was examined. Figure 4 shows the partial amino acid sequence of the factor IX-activating protein, which was obtained by the amino acid analysis. Databank analysis suggested that the protein was elastase. The molecular weight of the factor IX-activating protein of the present invention was approximately 29kDa.

### (2) Effect of shear rate on activation of factor IX

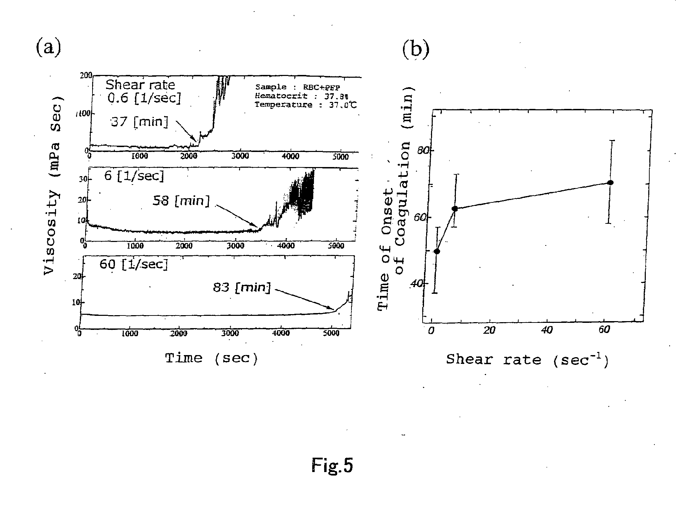

The effect of shear rate on coagulation of RBCs/PFP system was measured. Figure 5 (a) shows the results. An aqueous solution of calcium chloride was added to a sample to start coagulation, and then the sample was added to a gap of the cone-plate. Subsequently, the plate was rotated, thereby loading a certain shear rate to the sample. Viscosity increases when coagulation begins as shown in the figure. Thus, the time at which viscosity started to increase, was determined as the time of onset of coagulation, as shown with arrows in the figure. Figure 5 (b) shows the relation between the time of onset of coagulation and the shear rate. The time of onset of coagulation was significantly delayed as shear rate increased.

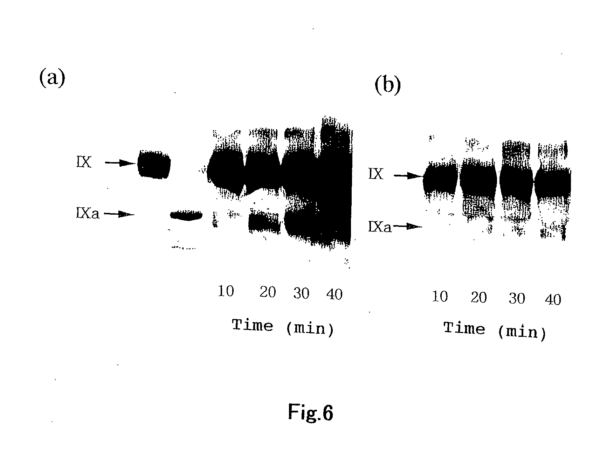

In order to examine the effect of shear rate on activation of factor IX by erythrocyte membrane, erythrocytes were washed and added to a solution containing factor IX, followed by loading a certain shear rate for a certain period of time using a cone-plate viscometer. After loading, the degree of activation of factor IX was examined by SDS-PAGE and Western blot method. As shown in Fig. 6, at a shear rate of 0.1 sec⁻¹, activation of factor IX started within 20 to 30 minutes, and at a rate of 50 sec⁻¹, almost no activation of factor IX occurred.

### (3) Effect of hematocrit (Ht) on coagulation of whole blood and RBCs/PFP system

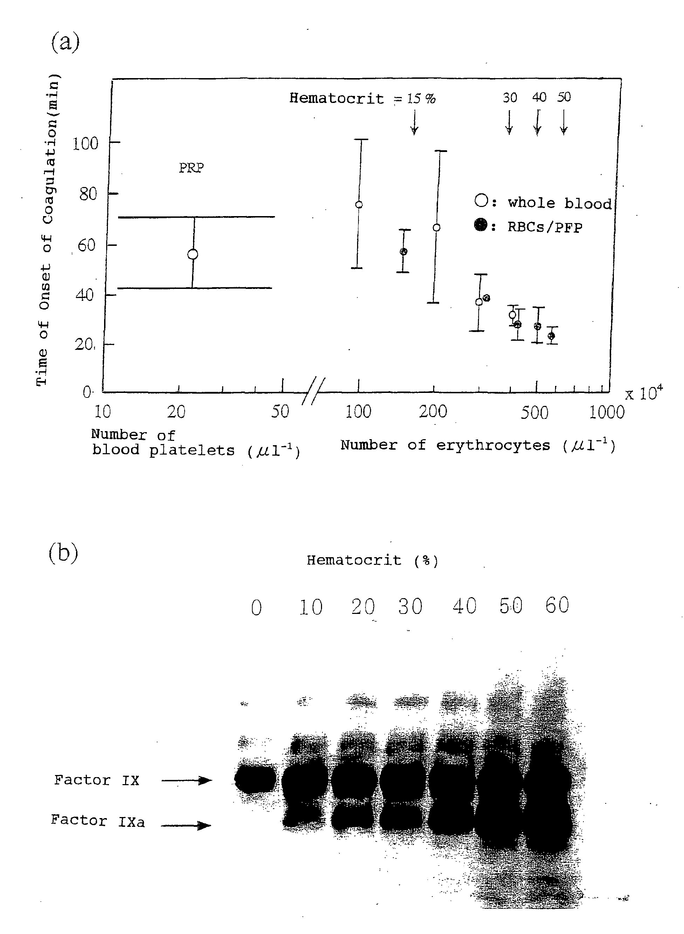

Effect of hematocrit (Ht) on coagulation of whole blood and RBCs/PFP system was measured. Figure 7(a) shows the results. In the whole blood and RBCs/PFP system, the time of onset of coagulation was significantly reduced as hematocrit increased. For platelet rich plasma (PRP), the time of onset of coagulation ranged from 40 to 70 minutes, and was independent of the number of platelets. The effect of hematocrit on activation of factor IX was examined by SDS-PAGE and Western blot method. As shown in Fig. 7(b), activation of factor IX was significantly accelerated as hematocrit increased.

### (4)The time of onset of coagulation of whole blood and RBCs/PFP for normal subjects (humans), diabetics and pregnant women

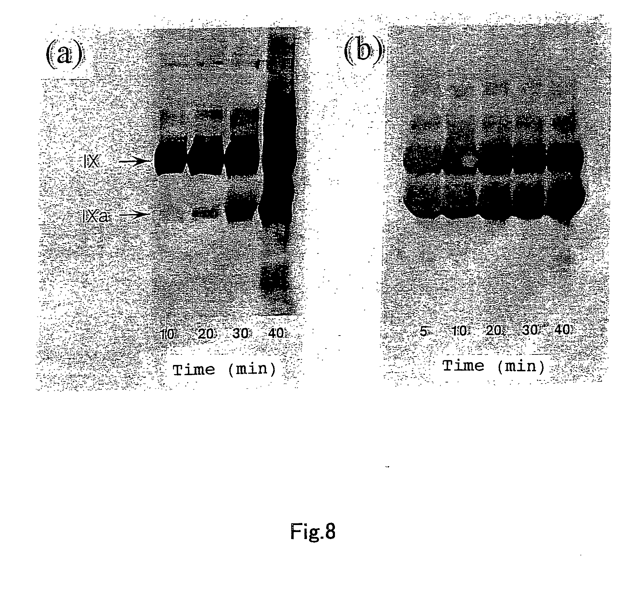

The time of onset of coagulation of whole blood and RBCs/PFP for normal subjects (humans), diabetics and pregnant women was measured. The results were shown in Table 1. Both the time of onset of coagulation of the whole blood and RBCs/PFP in diabetics and normal pregnant women were shorter than that of normal subjects. Activation of factor IX by the erythrocytes from normal subjects and diabetics was examined by SDS-PAGE and Western blot method. As shown in Fig. 8, factor IX was activated faster in the erythrocytes from diabetics compared to normal subjects. Factor IX was activated faster by the erythrocytes from normal pregnant women than those from normal subjects, as with those from diabetics.

**Table 1**

| The time of onset of coagulation of whole blood and RBCs/PFP in normal subjects, diabetics, and normal pregnant women | | |
|---|---|---|
| Blood (Number of experiments) | Time of onset of coagulation (min.) | |
| | Whole blood | RBCs/PFP |
| Normal subject (n>50) | 31.2±6.6 | 30.5±2.9 |
| Diabetic (n=11) | 24.1±6.2 | 23.3±7.0 |
| Normal pregnant woman (n=14) | 16.6±6.4 | 20.7±4.8 |

### (5) Dynamics of erythrocyte aggregation and thrombus formation in an aneurysmal flow-pass model

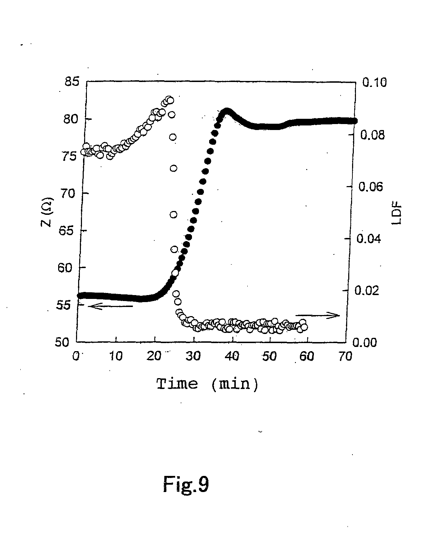

In order to confirm that thrombus formation can be detected by measuring impedance, impedance in the course of blood coagulation was measured under conditions without blood flow. Figure 9 shows the result. For comparison, changes in logarithmic damping factor (LDF) were measured using a damped oscillation rheometer. Figure 9 shows the results. Impedance significantly increased corresponding to changes in LDF in the course of thrombus formation.

In order to measure the course of thrombus formation under conditions with blood flow, experiments for visualizing a flow using micro-beads were conducted. In these experiments, conditions when blood flows and conditions when blood does not flow inside an aneurysm, were sought. In a system using RBCs/PFP samples, thrombus was formed only inside the aneurysm in which blood stagnation occurred.

### C. Discussion

### (1) Factor IX-activating protein

Factor IX, which is present in an erythrocyte membrane, was purified, suggesting that factor IX-activating protein present in erythrocyte membrane is quite likely to be an elastase.

Elastase is so named because it has an action of decomposing a protein, elastin, which is present in the tendon of an animal. Elastases are known to be distributed over a various sites in a body, such as in leukocytes, platelets, and spine (Anderssen T et al., Human leukocyte elastase and cathespin G inactivate factor VII by limited proteolysis. Thromb. Haemost 70: 414-417, 1993; and Kawamata M et al., Acute Pulmonary edema associated with transfusion of packed red blood cells. Intensive Care Med. 21: 443-446, 1995). Furthermore, elastase is a member of the serine protease family, which contains various proteases including digestive enzymes, such as chymotrypsin and trypsin, and thrombin which is involved in blood coagulation (Keiko Nakamura et al.,; Protein Structure, "Cell Molecular Biology" (No. 1), Kyo-iku-sha, pp. 111-127, 1985). These enzymes are quite similar to each other in their structure, in which the same amino acids compose approximately 40% of the enzyme. The enzymes have active serine residues, but are known to have totally different functions from each other. The changes in amino acids which correspond to the differences of the enzymes may be the result of evolutionary selection. That is, enzymes that have changed their properties concerning substrate specificity and regulation were selected through evolution.

### (2) Effect of shear rate on activation of factor IX

There are many risk factors in expression of blood clot. Risk factors relating to rheology are, for example, blood stagnation, erythrocyte aggregation, and increased hematocrit (Ernst E: Rheology of the post-thrombotic syndrome. J. des Maladies Vasculaires 17 : 93-96, 1992; and Mammen EF: Pathogenesis of venous thrombosis. Chest 102; 640-644, 1992). As shown in Fig. 5, the time of onset of coagulation of RBCs/PFP was significantly delayed as the shear rate increased. Moreover, as shown in Fig. 6, at high shear rates as high as 0.6 sec⁻¹ and 50sec⁻¹, factor IX was not activated by erythrocyte membranes. It has been known that at a shear rate in the range of 0 to 5 sec⁻¹, erythrocytes form aggregate structures, but a further increase in shear rate results in disruption of erythrocyte aggregation with the increased shear rate (Chien S; Shear dependence of effective cell volume as a determinant of blood viscosity. Science 168 : 977-979, 1970). Accordingly, activation of factor IX by erythrocytes is thought to be related to formation of an erythrocyte aggregation structure. Factor IX entrapped within an aggregate of erythrocytes is assumed to bind with a factor IX-activating protein and to cause activation reaction on the membrane surface. With a large shear rate, each erythrocyte membrane surface is constantly exposed to shear stress so that binding of factor IX to the membrane surface would be prevented.

### (3) Effect of hematocrit on activation of factor IX

Dehydration during sleep or after taking a bath is thought to be a risk factor of thrombus formation, since it causes increased hematocrit (Yasaka M et al., Hypercoagulability in the left atrium: Part II: Coagulation factors. J. Heart Valve Dis., 2, 25-34, 1993). Increased hematocrit reduces blood fluidity, and also increases the concentration of factor IX-activating proteins, allowing coagulation to occur easier.

### (4) Activation of factor IX by erythrocytes of normal human subjects and thrombophiliac human subjects

Blood of pregnant women and of diabetics and the like is known to be generally in a hypercoagulable state (Kaibara M et al., Effects of red blood cells on the coagulation of blood in normal and preeclamptic pregnancies. Am J. Obstet. Gynecol., 180, 402-405, 1999, Lupu C et al., Enhanced prothrombin and intrinsic factor X activation on blood platelets from diabetic patients. Thromb. Haemost. 70, 579, 1993). However, the reason remains unknown. The results shown in Table 1 and Fig. 8 suggest that factor IX is activated faster by erythrocytes of pregnant women and diabetics. In other words, it suggests a possibility that the concentration of factor IX-activating proteins and the properties and structure of erythrocyte membranes which provide reaction sites, are involved in activation of factor IX.

### (5) Dynamics of erythrocyte aggregation and thrombus formation in an aneurysm model

LDF in Fig. 9 is a physical quantity which shows blood fluidity. Coagulation starts upon the start of a sudden decrease in LDF. This time almost coincides with a time at which impedance started to change, suggesting that dynamics of thrombus formation can be analyzed by the impedance measurement method. However, a method for separating thrombus formation from erythrocyte aggregation needs to be studied in the future, since impedance varies depending on erythrocyte aggregation and precipitation.

### Example 2:

### (1) Cleavage site of factor IX activated by the enzyme

HEPES buffer containing the enzyme extracted in Example 1(1) (final concentration: 100 U/ml) and factor IX (about 0.2 *µ*g/ml) was incubated at 37°C for 1 hour. In order to determine the cleavage site of N-terminal of the heavy chain of factor IX activated by the extracted enzyme, the activated factor IX was transferred to PVDF membrane and was stained with Coomassie blue. The band corresponding to factor aβ was analyzed by a protein sequencer.

For determination of the cleavage site of C-terminal of light chain, factor IX was cut by an extracted enzyme, and was reduced with dithiothoreitol and was alkylated with acetic iodide. Then, heavy chain is separated and removed, and light chain was treated with endoprotease Asp-N for 2 hours. The treated fragment was subjected to mass analysis to determine the cleavage site at C-terminal.

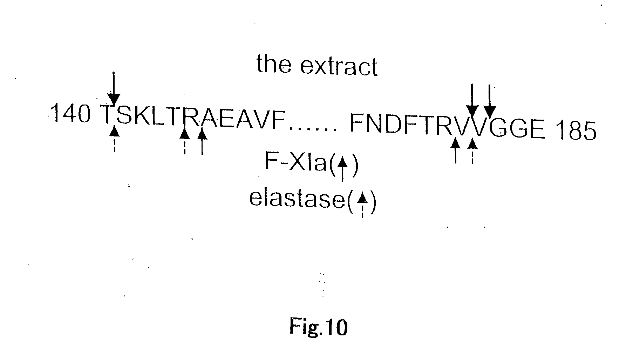

The cleavage site is shown in Fig. 10. As is understood from Fig. 10, the cleavage sites by the enzyme extracted in Example 1 (1) are different from those of activating factor XI (XIa). Also, two cleavage sites are the same in the extracted enzyme and elastase.

### (2) Evaluation of activity of enzyme on erythrocyte membrane (2-A) Observation with fluoromicroscope

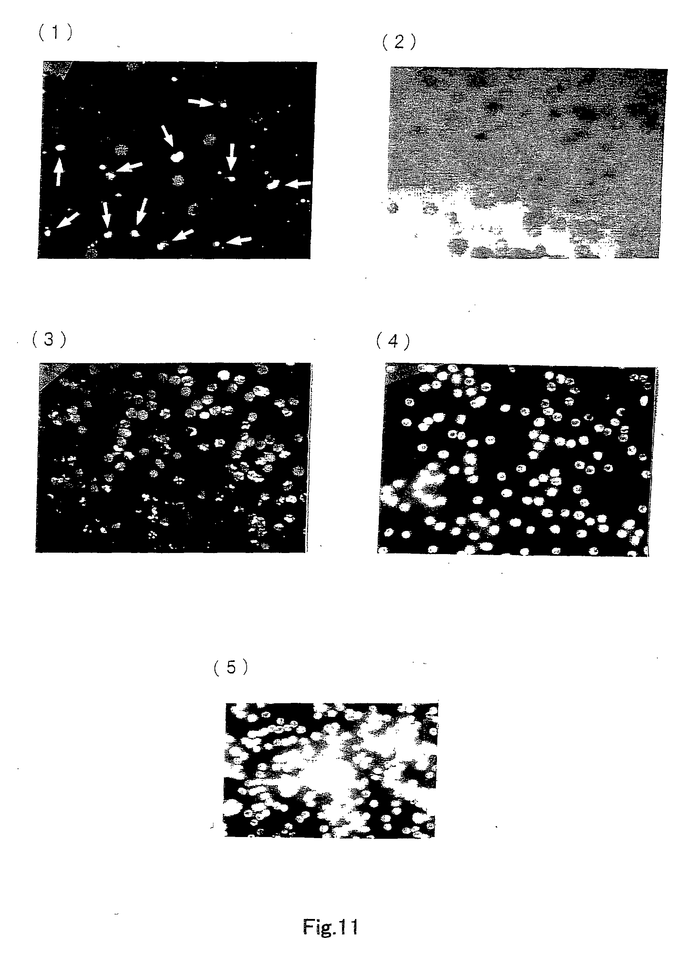

10 *µ* l of 1.5 *µ* M synthetic substrate (Suc(OMe)-Ala-Ala-Pro-Val-MCA) specific to the enzyme of the present invention (enzyme extracted in Example 1(1)) was added to 40 *µ*l of blood sample on polypropylene plate, and the plate was covered with cover glass and incubated at room temperature for 30 minutes. The fluorescence from the sample was observed under a fluoromicroscope (Model AX70, Olympus) at λex=380nm and λ em=460nm. The results are shown in Fig. 11. In Fig. 11, fluorescence is observed if a synthetic substrate is cut with an enzyme on erythrocyte membrane to release AMC.

In Fig. 11, each figure shows the following samples.
(1) In the case of platelet rich plasma (PRP) (number of leukocytes: 4000 cells/*µ*l); an arrow represents leukocytes. The enzyme of the present invention is considered to be present in leukocytes since a fluorescence is observed in leukocytes.
(2) In the case where AMC was added onto erythrocytes; AMC does not adsorbed onto erythrocytes.
(3) In the case of normal erythrocytes;
(4) In the case of erythrocytes of diabetics; and
(5) In the case of reticulocytes
(Note: the number of erythrocytes is about 240,000 cells/*µ*l which is about 1/20 of the number under the physiological condition. The number of leukocytes is that under the physiological condition. Therefore, the onset of coagulation is considered to be controlled an enzyme present on erythrocyte membrane.)

### (2-B) Evaluation by measurement of fluorescent intensity of blood sample

Erythrocytes were mixed with HEPES buffer containing 22.5 nM synthetic substrate (Suc(OMe)-Ala-Ala-Pro-Val-MCA) (Hematocrit: 1.5%), and the mixture was incubated at 37°C for 30 minutes. After reaction, the mixture was fully stirred, and erythrocyte suspension was centrifuged. The resultant supernatant was diluted 20 times, and the fluorescent intensity of the solution was measured( λex=380nm, λem=460nm). The results are shown below.
Human normal erythrocytes
Average 16.4±7.2 (n=10)
Maximum 29.2
Minimum 9.0
Reticulocytes
390, 534

The fluorescent intensity of reticulocytes was 20-30 times of that of normal erythrocytes. As described above, the cleavage sites by the extracted enzyme are different from those of elastase. These results suggests that the factor IX activating enzyme present on erythrocyte membrane was not derived from leukocytes but was produced by gene coding in the course of erythrocyte maturation.

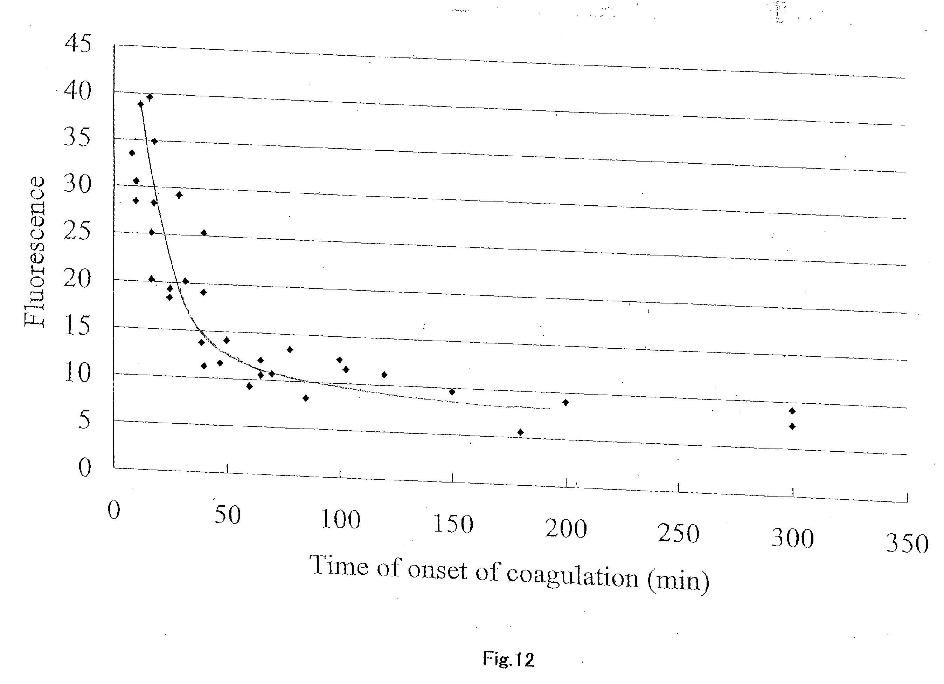

Figure 12 shows the relation between the fluorescent intensity and the time of onset of coagulation in the sample (RBCs/PFP) free from platelets and leukocytes. The fluorescent intensity was measured by a method described in Example 2 (2-B) "Evaluation by measurement of fluorescent intensity of blood sample", and the time of onset of coagulation was measured by a method described in Example 1, A. Method, "(2) Measurement of the time of onset of blood coagulation".

As is understood from Fig. 12, the fluorescent intensity becomes stronger as the time of onset of coagulation is shorter.

### Industrial applicability

The mechanism of the intrinsic coagulation reaction, which is caused by activation of blood coagulation factor IX by erythrocyte membranes, is partially revealed by the present invention. The blood coagulation factor IX-activating protein, and its antibody and labeled antibody according to the present invention are useful in diagnosis, prevention and/or treatment for diseases associated with blood coagulation, such as thrombosis.

## Claims

1. A blood coagulation factor IX-activating protein derived from a mammal, having the following properties:
(1) the protein acts on blood coagulation factor IX to activate said factor;
(2) the activity of the protein is inhibited in the presence of an α1-protease inhibitor or soybean trypsin inhibitor;
(3) the protein is present in erythrocyte membrane;
(4) the protein has a molecular weight of approximately 29 kDa as measured by SDS-PAGE.

2. The blood coagulation factor IX-activating protein according to claim 1 which cuts the amino acid sequence of the blood coagulation factor IX between 140th threonine and 141th serine, between 181th valine and 182th valine, and between 182th valine and 183th glycin.

3. The blood coagulation factor IX-activating protein according to claim 1 which comprises the following partial amino acid sequence:

Ile-Val-Gly-Gly-Arg-Arg-Ala-Arg-Pro-His-Ala-Trp-Pro-Phe-
Met-Val-Ser-Leu

4. The blood coagulation factor IX-activating protein according to claim 1 which is derived from human.

5. The blood coagulation factor IX-activating protein according to claim 1 which is purified by disrupting erythrocytes, extracting with a surfactant, and subjetcing the extract to anion exchange chromatography and heparin affinity chromatography.

6. A medicine which comprises a blood coagulation factor IX-activating protein according to claim 1.

7. The medicine according to claim 6 which is used for treatment and/or prevention of diseases associated with abnormal blood coagulation.

8. An agent for activating blood coagulation factor IX which comprises a blood coagulation factor IX-activating protein of claim 1.

9. An antibody or fragment thereof which recognizes a blood coagulation factor IX-activating protein of claim 1.

10. The antibody or fragment thereof according to claim 9 wherein the antibody is a monoclonal antibody.

11. A medicine which comprises an antibody or fragment thereof of claim 9.

12. The medicine according to claim 11 which is used for treatment and/or prevention of diseases associated with abnormal blood coagulation.

13. A labeled antibody or fragment thereof which recognizes a blood coagulation factor IX-activating protein of claim 1.

14. A medicine which comprises a labeled antibody or fragment thereof of claim 13.

15. The medicine according to claim 14 which is a diagnostic drug for diseases associated with blood coagulation.

16. A method for detecting a blood coagulation factor IX-activating ability which comprises detecting or measuring a blood coagulation factor IX-activating protein of claim 1 in a biological sample.
